# EUROPÄISCHE PATENTANMELDUNG

(11) **EP 1 145 984 A1**
(43) Veröffentlichungstag der Anmeldung: **17.10.2001**
(21) Anmeldenummer: 01108799.6
(22) Anmeldetag: 07.04.2001
(51) Int. Cl.: B65D 77/24, B65D 73/00

(54) **Applikationshilfseinrichtung für Wund- oder Gewebekleber**

(30) Priorität: 12.04.2000 DE 20006716 U
(71) Anmelder: Resorba Chirurgisches Nahtmaterial Franz Hiltner GmbH & Co., 90443 Nürnberg (DE)
(72) Erfinder: Hiltner, Claus M., 90408 Nürnberg (DE)
(74) Vertreter: Schneck, Herbert, Dipl.-Phys., Dr.

(57) **Zusammenfassung**

Bei einer Applikationshilfseinrichtung (1) für Wund- oder Gewebekleber ist zur Gewährleistung einer sterilen Aufbewahrung und Applikation einerseits und einer Mehrfachverwendbarkeit des Klebers andererseits vorgesehen, daß in einer geschlossenen, sterilen Packung eine Pipette (5) und eine Vertiefung (3) zur Einbringung des Klebers vorgesehen sind.

## Beschreibung

Die Erfindung richtet sich auf eine Applikationshilfseinrichtung für Wund- oder Gewebekleber.

Der Erfindung liegt die Aufgabe zugrunde, eine solche Hilfseinrichtung so auszugestalten, daß einerseits eine sterile Aufbewahrung und Applikation gewährleistet ist und andererseits eine Mehrfachverwendbarkeit des Klebers erreicht wird.

Diese Aufgabe wird erfindungsgemäß dadurch gelöst, daß bei einer derartigen Applikationshilfseinrichtung in einer geschlossenen, sterilen Packung eine Pipette und eine Vertiefung zur Einbringung des Klebers vorgesehen sind.

Dementsprechend ist es möglich, den Kleber aus einem Vorratsbehälter, insbesondere einer Phiole, in der gewünschten Menge in die Vertiefung zu dosieren und mit Hilfe der sterilen Pipette zu applizieren. Der Klebervorrat kann danach wieder geschlossen und aufbewahrt werden.

In weiterer Ausgestaltung der Erfindung ist vorgesehen, daß die Packung als Blisterpackung ausgebildet ist, wobei die Vertiefung im Boden vorzugsweise einstückig mit der Blisterpackung ausgebildet ist, beispielsweise als tiefgezogenes Kunststoffteil.

Weiterhin kann vorgesehen sein, daß im Boden des Blisters eine Klemmrinne für die Pipette angeordnet ist, so daß diese definiert festgelegt ist, bis sie zur Applikation benötigt wird.

Dabei kann die Klemmrinne etwa diagonal auf einem im wesentlichen rechteckigen Bodenteil angeordnet sein, so daß einerseits Platz für die vorgesehene Vertiefung bleibt und andererseits die Längenerstreckung der Verpackung minimiert wird.

Nachfolgend wird die Erfindung anhand eines bevorzugten Ausführungsbeispiels in Verbindung mit der Zeichnung näher erläutert. Dabei zeigen:

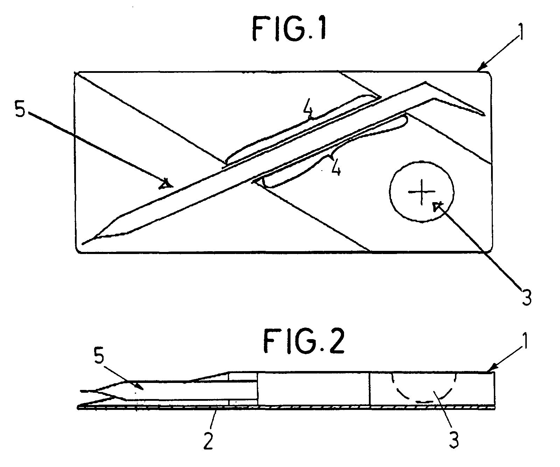

- Fig. 1: eine Aufsicht auf eine erfindungsgemäße Applikationshilfseinrichtung und
- Fig. 2: einen Schnitt durch den Bereich der Vertiefung.

Eine in der Zeichnung dargestellte Applikationshilfseinrichtung 1 ist als Blister aus Kunststoff ausgebildet, welches in einen Beutel aus semipermeablen Materialien eingebracht wird.

In dem Bodenteil 2 ist eine Ausnehmung 3 für den Kleber ausgebildet sowie eine Klemmrinne 4 für eine Pipette 5. Die Blisterpackung und die Pipette 5 werden in dem verschlossenen Beutel sterilisiert und somit in einem sterilen Zustand aufbewahrt.

Zur Applikation wird der Beutel aufgezogen und aus einer Phiole Kleber in die Ausnehmung 3 eingebracht. Mit Hilfe der Pipette 5 wird eine bestimmte Menge von Kleber aus der Vertiefung aufgenommen und auf die Wunde appliziert. Die Phiole wird anschließend wieder geschlossen und zum mehrfachen nachfolgenden Gebrauch verwahrt, wohingegen die Pipette 5 und der Blister ebenso wie der Beutel weggeworfen werden.

## Patentansprüche

1. Applikationshilfseinrichtung für Wund- oder Gewebekleber, **dadurch gekennzeichnet, daß** in einer geschlossenen, sterilen Packung eine Pipette (5) und eine Vertiefung (3) zur Einbringung des Klebers vorgesehen sind.

2. Applikationshilfseinrichtung nach Anspruch 1, **dadurch gekennzeichnet, daß** die Packung als Blister ausgebildet ist

3. Applikationshilfseinrichtung nach Anspruch 2, **dadurch gekennzeichnet, daß** die Vertiefung im Boden (2) des Blisters einstückig mit dieser ausgebildet ist.

4. Applikationshilfseinrichtung nach Anspruch 3, **dadurch gekennzeichnet, daß** im Boden (2) der Blisterpackung eine Klemmrinne (4) für die Pipette (5) angeordnet ist.

5. Applikationshilfseinrichtung nach Anspruch 1, **dadurch gekennzeichnet, daß** die Klemmrinne (4) etwa diagonal auf einem im wesentlichen rechteckigen Bodenteil (2) angeordnet ist.
